# EUROPEAN PATENT SPECIFICATION

(11) **EP 2 803 330 B1**
(45) Date of publication and mention of the grant of the patent: **05.09.2018**
(21) Application number: 14168887.9
(22) Date of filing: 19.05.2014
(51) Int. Cl.: A61C 3/00, A61B 90/90

(54) **A dental instrument and a method for manufacturing the same**
Zahnärztliches Instrument und Verfahren zu seiner Herstellung
Instrument dentaire et procédé de fabrication de celui-ci

(30) Priority: 17.05.2013 FI 20135531
(43) Date of publication of application: 19.11.2014
(60) Divisional application: 18182092.9
(73) Proprietor: LM-Instruments Oy, 21600 Parainen (FI)
(72) Inventor: Lehtonen, Kari, 21601 Parainen (FI)
(74) Representative: Finnpatent Oy

(56) References cited:
- US-A1- 2007 159 336
- US-A1- 2012 107 770
- US-B1- 6 322 362

## Description

### Field of the invention

The invention relates to a dental instrument and to a method for manufacturing the same.

### Background

In many cases, authorities and actors of the dental field want to have an infallible and traceable solution to follow dental instruments so as be able to trace disinfection, sterilization, reparation, and other operations directed to or carried out with the dental instruments under consideration. Nowadays, users do not typically have the time and willingness to generate reports manually because of the related workload. In addition, there is a risk of errors with manual data recording and identification of dental instruments, which prevents regarding the manually recorded data as an irrefutable proof of what has been done and what has been not done.

A known solution uses tags including a radio frequency identifier "RFID". The tag is glued on the dental instrument to be identified. It is however challenging to provide a glue joint that produces a reliable long term adhesion of the tag on the dental instrument in cases where the dental instrument is exposed to operations such as sterilization, disinfection, and/or ultrasonic baths.

WO2008062387 describes a dental instrument comprising a radio frequency identifier. The radio frequency identifier is embedded in a polymer sheet that is attached on a surface of the handle of the dental instrument. The polymer sheet that includes the radio frequency identifier can be, for example, wrapped around the handle of the dental instrument. In order to obtain an even surface, it is possible to provide the dental instrument with a recess corresponding in size to the polymer sheet. In an advantageous embodiment described in WO2008062387, the polymer sheet comprises two layers between which the radio frequency identifier is positioned. The two layers are made of materials having different hardness. The layer of the harder material is against the dental instrument in order to obtain a better adhesion. The softer material protects the radio frequency identifier against external impacts. It may be, however, in some circumstances hard to guarantee that the polymer sheet remains firmly attached to the dental instrument. Furthermore, it may be hard to guarantee that no slits are left between the polymer sheet and the handle of the dental instrument. The slits are undesirable because, in some circumstances, they may collect impurities. US 2007/0159336 A1 relates to a medical instrument comprising a radio frequency identifier overmolded on the metallic core of the instrument, wherein the radio frequency identifier operates in the 868-927 MHz frequency band, corresponding to a wavelength ranging from 323 to 345 mm.

### Summary

The following presents a simplified summary in order to provide a basic understanding of some aspects of various embodiments of the invention. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

In accordance with the invention, there is provided a new method for manufacturing a dental instrument that comprises operative portions for operations according to the purpose of use of the dental instrument, a body portion between the operative portions and mechanically connecting to the operative portions, and a radio frequency identifier.

The manufacturing method according to the invention comprises:
- placing the radio frequency identifier on the body portion of the dental instrument, and
- providing a tubular handle portion to surround the radio frequency identifier and at least a part of the body portion by casting the tubular handle portion on the radio frequency identifier and the body portion so that the material of the tubular handle portion gets adhered to the material of the body portion and so that the radio frequency identifier is positioned on the body portion and in between the tubular handle portion and the body portion after the casting.

The body portion is made of metal and a length of the dental instrument is about 146 mm - 210 mm, and the radio frequency identifier is arranged to use wave length of 300 mm.

The benefit of casting the tubular handle portion on the dental instrument so that the material of the tubular handle portion is adhered to the material of the body portion is that there are no such slits between the tubular handle portion and the body portion which would collect impurities, and thus it is easier to keep the dental instrument hygienic. Therefore, the method according to the invention provides a dental instrument where the tubular handle portion acts not only as the handle portion for providing a gripping surface of the dental instrument but also as at least a part of a fixation system that:
- attaches the radio frequency identifier to the dental instrument, and furthermore
- is easy to keep hygienic.

The tubular handle portion is advantageously made of material whose electrical resistivity is sufficiently high so that the operation of the radio frequency identifier is not hindered. The electrical resistivity is advantageously at least 10⁻³ Ωm at 20°C. More advantageously, the tubular handle portion is made of electrically non-conductive material.

In a dental instrument according to an advantageous and exemplifying embodiment of the invention, the material of the tubular handle portion is mechanically softer than the material of the body portion. Thus, the body portion can be arranged to provide the required mechanical stiffness and the tubular handle portion can be arranged to enable a user to have a good grip on the dental instrument with a relatively small pressing force.

In accordance with the invention, there is provided also a new dental instrument that comprises:
- operative portions for operations according to the purpose of use of the dental instrument,
- a body portion between the operative portions and mechanically connecting to the operative portions,
- a radio frequency identifier on the body portion and capable of being read from a distance of the dental instrument and capable of storing information, and
- a tubular handle portion provided to surround the radio frequency identifier and at least a part of the body portion so that the radio frequency identifier is positioned in between the tubular handle portion and the body portion, the tubular handle portion being cast on the radio frequency identifier and the body portion so that the material of the tubular handle portion is adhered to the material of the body portion.

The body portion is made of metal and a length of the dental instrument is about 146 mm - 210 mm, and the radio frequency identifier is arranged to use wave length of 300 mm.

A number of non-limiting and exemplifying embodiments of the invention are described in accompanied dependent claims.

Various non-limiting and exemplifying embodiments of the invention both as to constructions and to methods of manufacturing, together with additional objects and advantages thereof, will be best understood from the following description of specific exemplifying embodiments when read in connection with the accompanying drawings.

The verbs "to comprise" and "to include" are used in this document as open limitations that neither exclude nor require the existence of unrecited features. The features recited in depending claims are mutually freely combinable unless otherwise explicitly stated. Furthermore, it is to be understood that the use of "a" or "an", i.e. a singular form, throughout this document does not exclude a plurality.

### Brief description of the figures

Exemplifying embodiments of the invention and their advantages are explained in greater detail below in the sense of examples and with reference to the accompanying drawings, in which:
figures 1a and 1b illustrate a dental instrument according to an exemplifying embodiment of the invention,
figures 2a, 2b and 2c show cross-sections of dental instruments according to exemplifying embodiments of the invention, and
figure 3 shows a flowchart of a method according to an exemplifying embodiment of the invention for manufacturing a dental instrument.

### Description of exemplifying embodiments

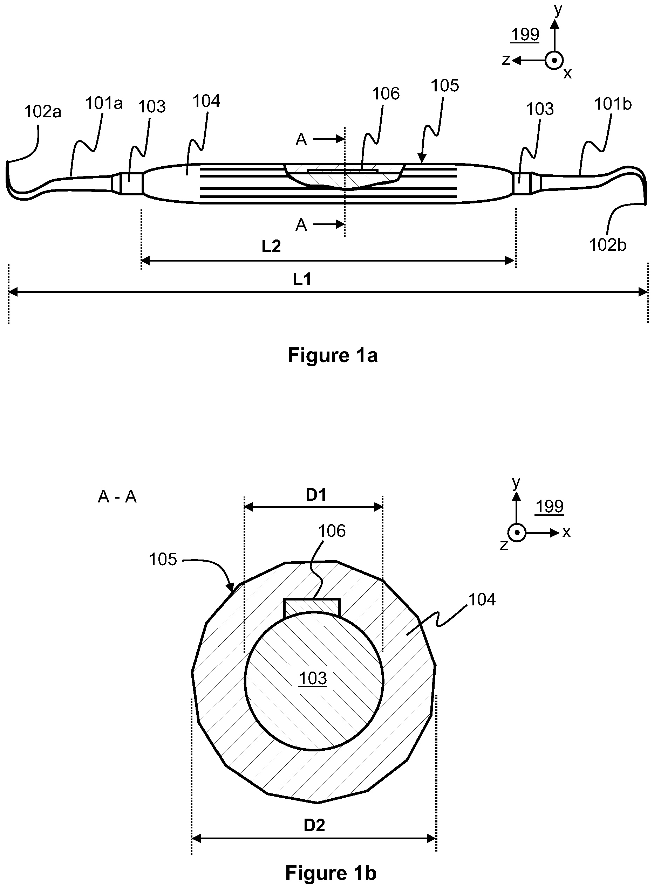

Figure 1a shows a partial section view of a dental instrument according to an exemplifying embodiment of the invention. Figure 1b shows a view of a section taken along the line A - A shown in figure 1a. The section plane is parallel with the xy-plane of a coordinate system 199. The dental instrument illustrated in figures 1a and 1b is obtainable with a method according to an exemplifying embodiment of the invention. The dental instrument is suitable for e.g. removing dental calculus. The dental instrument comprises operative portions 101a and 101b that have tip portions 102a and 102b for performing the operations according to the purpose of use of the dental instrument. The tip portions can operate as cutting edges for mechanical treatment. In the exemplifying case illustrated in figure 1a, the operating portions 101a and 101b are bent in order to facilitate the dental instrument reaching the target in the patient's mouth. Typically, sharply bent operating portions are suitable for use e.g. in the molar area often comprising targets difficult to reach. On the other hand, operating portions intended for working in the incisor area are typically more gently bent. It is worth noting that the operating portions are not necessarily sharp but one or both of them can be designed to be suitable for e.g. modelling tooth filling material in the patient's mouth. The dental instrument comprises an elongated body portion 103 that constitutes a mechanical support core of the dental instrument and is mechanically connected to the operative portions 101a and 101b as illustrated in figure 1a. In the exemplifying case illustrated in figures 1a and 1b, the dental instrument comprises operative portions 102 at its both ends. The dental instrument comprises a radio frequency identifier "RFID" 106 capable of being read from a distance of the dental instrument and capable of storing information. The information may contain for example identifying information identifying the dental instrument as an individual object from among similar dental instruments and/or information indicating e.g. a date of manufacture of the dental instrument, the manufacturer of the dental instrument and/or some other information related directly or indirectly to the dental instrument. The dental instrument further comprises a tubular handle portion 104 that has been mold cast to surround the radio frequency identifier 106 and at least a part of the body portion 103 so that the material of the tubular handle portion 104 is adhered to the material of the body portion. The above-mentioned radio frequency identifier 106 is positioned between the body portion 103 and the tubular handle portion 104 as illustrated in figures 1a and 1b. The benefit of the structure where the material of the tubular handle portion 104 is adhered to the material of the body portion 103 is that there are no such slits between the tubular handle portion 104 and the body portion 103 which could collect impurities, and thus the dental instrument is easier to keep hygienic. Thus, the tubular handle portion 103 acts not only as the handle portion for providing a gripping surface 105 of the dental instrument but also as at least a part of a fixation system attaching the radio frequency identifier 106 to the dental instrument. A dental instrument of such construction is easy to keep hygienic.

The tubular handle portion 104 is advantageously made of material whose electrical resistivity is sufficiently high so that the operation of the radio frequency identifier 106 is not hindered. The electrical resistivity is advantageously at least 10⁻³ Ωm at 20°C. More advantageously, the tubular handle portion 104 is made of electrically non-conductive material.

In a dental instrument according to an exemplifying embodiment of the invention, the material of the tubular handle portion 104 is mechanically softer than the material of the body portion 103. The body portion 103 can be arranged to provide the required mechanical stiffness whereas the tubular handle portion 104 can be arranged to enable a user to have a good grip on the dental instrument with a relatively small pressing force.

In a dental instrument according to an exemplifying embodiment of the invention, the material of the body portion 103 is one whose modulus of elasticity is at least 1000 MPa. Examples of materials fulfilling this criterion include metals such as aluminum, titanium and magnesium, stainless steels and also some stiff polymers. The body portion 130 must be stiff enough so that upon use of the dental instrument, when forces act on the dental instrument tending to bend it, the radio frequency identifier 106 does not get loose or even break.

In a dental instrument according to an exemplifying embodiment of the invention, the material of the tubular handle portion 104 is preferably silicone or other suitable elastomer, not only because of its properties in view of gripping the dental instrument but also since these materials are flexible and soft enough to protect the radio frequency identifier 106 against impacts.

In a dental instrument according to an exemplifying embodiment of the invention, the material of the tubular handle portion 104 is silicone or other suitable elastomer and the material of the body portion 103 is one of the following: aluminum, steel, polyether-ether-ketone "PEEK", polyphenylene-sulfide "PPS", polysulfone "PSU", polyamide "PPSU", polyetherimide "PEI", polyamide "PA", polypropene "PP", polycarbonate "PC".

In a dental instrument according to an exemplifying embodiment of the invention, the total length L1 of the dental instrument is advantageously on the range 146 mm - 210 mm, the length of the tubular handle portion 104 is on the range 92 mm - 132 mm, the diameter D1 of a smallest circle through which the body portion 103 is capable of being threaded is on the range 4 mm - 6 mm, and the diameter D2 of a smallest circle through which the tubular handle portion 104 is capable of being threaded is on the range 8 mm - 12 mm. If the body portion 103 has a circular cross-section as in the case shown in figure 1b, the diameter D1 is the diameter of the cross-section of the body portion at its thickest point. Correspondingly, if the tubular handle portion 104 has a circular cross-section, the diameter D2 is the diameter of the cross-section of the tubular handle portion 104 at its thickest point.

When the body portion 103 is made of metal, the radio frequency used by the radio frequency identifier "RFID" 106 is selected so that the wave length of the radio waves is 300 mm, which is a wave length found to be extremely suitable for use in this context.

As an alternative to the structure of the dental instrument shown in figure 1a, the tubular handle portion 104 may be arranged to extend over the entire body portion 103 and to cover even a part of the operative portion 101a and/or a part of the operative portion 101b.

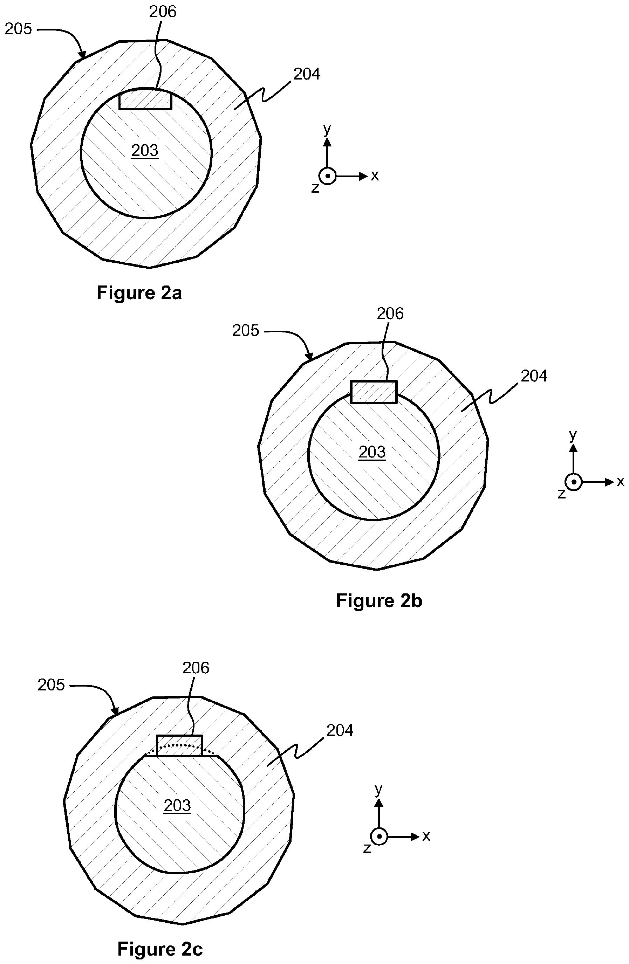

Figure 2a shows a cross-section of a dental instrument according to an exemplifying embodiment of the invention. The cross-section shown in figure 2a corresponds to the cross-section shown in figure 1b. The dental instrument comprises operative portions for performing the operations according to the purpose of use of the dental instrument, which operative portions are not shown in figure 2a, however. The dental instrument comprises a body portion 203 that constitutes a mechanical support core of the dental instrument and is mechanically connected to the operative portions. The dental instrument comprises a tubular handle portion 204 surrounding at least a part of the body portion 203 and constituting a gripping surface 205 of the dental instrument. The dental instrument further comprises a radio frequency identifier "RFID" 206 capable of being read from a distance of the dental instrument and capable of storing information. The radio frequency identifier 206 is positioned between the body portion 203 and the tubular handle portion 204 as illustrated in figure 2a. In this exemplifying case illustrated in figure 2a, the body portion 203 comprises a cavity for the radio frequency identifier 206.

Figure 2b shows a cross-section of a dental instrument according to an exemplifying embodiment of the invention. The cross-section shown in figure 2b corresponds to the cross-section shown in figure 1b. In the exemplifying case illustrated in figure 2b, both the body portion 203 and the tubular handle portion 204 comprise a cavity for the radio frequency identifier 206.

Figure 2c shows a cross-section of a dental instrument according to an exemplifying embodiment of the invention. The cross-section shown in figure 2c corresponds to the cross-section shown in figure 1b. In the exemplifying case illustrated in figure 2c, the body portion 203 comprises a flat facet for the radio frequency identifier 206. In one preferable embodiment, the facet could rather be described being a small cavity arranged on the surface of a basically cylindrical body portion 203. In one preferable embodiment, dimensions of the cavity correspond to those of the radio frequency identifier 206.

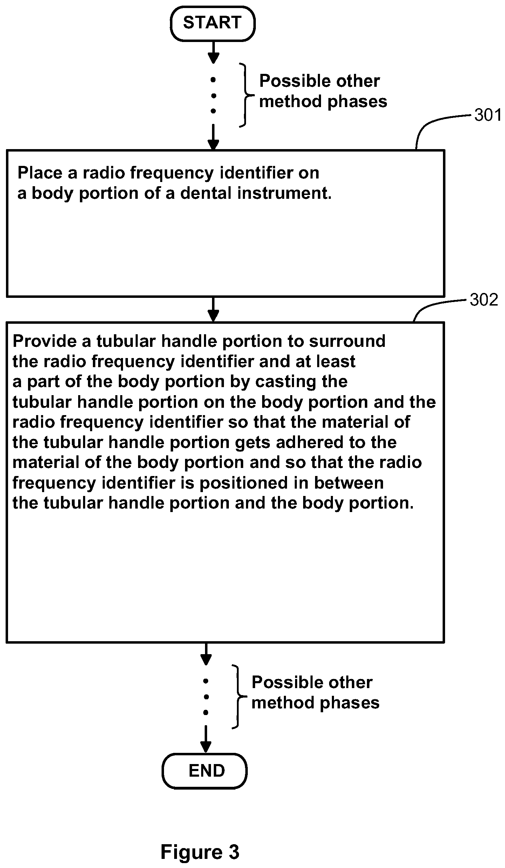

Figure 3 shows a flowchart of a method according to an exemplifying embodiment of the invention for manufacturing a dental instrument that comprises operative portions for operations according to the purpose of use of the dental instrument, a body portion between the operative portions and mechanically connecting to the operative portion, and a radio frequency identifier. The method comprises the following actions:
- action 301: placing the radio frequency identifier on the body portion of the dental instrument, and subsequently
- action 302: providing a tubular handle portion to surround the radio frequency identifier and at least a part of the body portion by casting the tubular handle portion on the radio frequency identifier and the body portion so that the material of the tubular handle portion gets adhered to the material of the body portion and so that the radio frequency identifier is positioned in between the tubular handle portion and the body portion after the casting.

In a method according to an exemplifying embodiment of the invention, placing of the radio frequency identifier on the body portion of the dental instrument comprises attaching the radio frequency identifier to the surface of the body portion with adhesive material.

In a method according to an exemplifying embodiment of the invention, placing of the radio frequency identifier on the body portion of the dental instrument comprises attaching the radio frequency identifier to the surface of the body portion with adhesive material.

In a method according to another exemplifying embodiment of the invention, the body portion of the dental instrument is arranged to comprise a cavity with dimension corresponding to those of the radio frequency identifier to ensure that the radio frequency identifier remains at its desired position during casting of the tubular hand portion on the dental instrument.

In order to sum up, preferable embodiments of the invention include a dental instrument which comprises an elongated handle portion (104, 204) and operative portions (101a, 101b) at the ends of the handle portion (104, 204) for operations according to one or more purpose of use of the dental instrument, and a body portion (103, 203) between the operative portions (101a, 101b) mechanically connecting to the operative portions (101a, 101b) and at least partially covered by the handle portion (104, 204), which dental instrument further comprises a radio frequency identifier (106, 206) comprising a component for storing information which is readable from a distance of the dental instrument so that said radio frequency identifier (106, 206) is covered by said handle portion (104, 204), the handle portion (104, 204) being cast on the radio frequency identifier (106, 206) and the body portion (103, 203) so that material of the tubular handle portion (104, 204) is adhered to material of the body portion (103, 203), whereby the radio frequency identifier is positioned on the body portion (103, 203) and in between the tubular handle portion (104, 204) and the body portion (103, 203), as well as a method for manufacturing a dental instrument comprising an elongated handle portion (104, 204) and operative portions (101a, 101b) at the ends of the handle portion (104, 204) for operations according to one or more purpose of use of the dental instrument, and a body portion (103, 203) between the operative portions (101a, 101b) mechanically connecting to the operative portions (101a, 101b) and at least partially covered by the handle portion (104, 204), which method comprises placing (301) the radio frequency identifier on the body portion of the dental instrument by providing (302) a tubular handle portion to surround the radio frequency identifier and at least a part of the body portion by casting the tubular handle portion on the radio frequency identifier and the body portion so that material of the tubular handle portion gets adhered to material of the body portion and so that the radio frequency identifier is positioned in between the tubular handle portion and the body portion after the casting.

According to the current understanding of the applicant, the various aspects and benefits of the invention are best achievable by a construction in which a tubular handle portion surrounds the radio frequency identifier and at least a part of the body portion of the dental instrument, in which the tubular handle portion is cast on the dental instrument so that material of the tubular handle portion is adhered to material of the body portion, in which the material of the tubular handle portion is silicone or some other elastomer which is mechanically softer than the material of the body portion, while the body portion whose modulus of elasticity is at least 1000 MPa constitutes a mechanical support core of the dental instrument, and wherein the body portion comprises a cavity for the radio frequency identifier.

The specific examples provided in the description given above should not be construed as limiting the applicability and/or the interpretation of the appended claims.

## Claims

1. A dental instrument comprising:
- an elongated handle portion (104, 204) and operative portions (101a, 101b) at the ends of the handle portion (104, 204) for operations according to one or more purpose of use of the dental instrument, and
- a body portion (103, 203) between the operative portions (101a, 101b) mechanically connecting to the operative portions (101a, 101b) and at least partially covered by the handle portion (104, 204),
wherein the dental instrument further comprises a radio frequency identifier (106, 206) which is readable from a distance of the dental instrument so that said radio frequency identifier (106, 206) is covered by said handle portion (104, 204), the handle portion (104, 204) being cast on the radio frequency identifier (106, 206) and the body portion (103, 203) so that material of the tubular handle portion (104, 204) is adhered to material of the body portion (103, 203), whereby the radio frequency identifier is positioned on the body portion (103, 203) and in between the tubular handle portion (104, 204) and the body portion (103, 203), **characterized in that** the body portion is made of metal and a length of the dental instrument (L1) is about 146 mm - 210 mm, and the radio frequency identifier (106, 206) is arranged to use wave length of 300 mm.

2. A dental instrument according to claim 1, wherein electrical resistivity of the material of the tubular handle portion is at least 10⁻³ Ωm at 20°C.

3. A dental instrument according to claim 1, wherein the material of the tubular handle portion is electrically non-conductive.

4. A dental instrument according to any of claims 1-3, wherein the material of the tubular handle portion is mechanically softer than the material of the body portion and the body portion constitutes a mechanical support core of the dental instrument.

5. A dental instrument according to any of claims 1-4, wherein modulus of elasticity of the material of the body portion is at least 1000 MPa.

6. A dental instrument according to any of claims 1-5, wherein the material of the tubular handle portion is silicone or some other elastomer.

7. A dental instrument according to any of claims 1-6, wherein the body portion (203) comprises a cavity for the radio frequency identifier.

8. A dental instrument according to claim 6, wherein the material of the body portion is one of the following: aluminum, steel, polyether-ether-ketone "PEEK", polyphenylene-sulfide "PPS", polysulfone "PSU", polyamide "PPSU", polyetherimide "PEI", polyamide "PA", polypropene "PP", polycarbonate "PC".

9. A method for manufacturing a dental instrument comprising an elongated handle portion (104, 204) and operative portions (101a, 101b) at the ends of the handle portion (104, 204) for operations according to one or more purpose of use of the dental instrument, and a body portion (103, 203) between the operative portions (101a, 101b) mechanically connecting to the operative portions (101a, 101b) and at least partially covered by the handle portion (104, 204), the method comprising:
- placing (301) a radio frequency identifier (106, 206) on the body portion of the dental instrument by providing (302) a tubular handle portion to surround the radio frequency identifier and at least a part of the body portion by casting the tubular handle portion on the radio frequency identifier and the body portion so that material of the tubular handle portion gets adhered to material of the body portion and so that the radio frequency identifier is positioned in between the tubular handle portion and the body portion after the casting,
**characterized in that** the body portion is made of metal and a length of the dental instrument (L1) is about 146 mm - 210 mm, and the radio frequency identifier (106, 206) is arranged to use wave length of 300 mm.

10. A method according to claim 9, wherein placing of the radio frequency identifier on the body portion of the dental instrument comprises attaching the radio frequency identifier to a surface of the body portion with adhesive material.

11. A method according to claim 10, wherein the body portion of the dental instrument is arranged to comprise a cavity with dimensions corresponding to those of the radio frequency identifier and the radio frequency identifier is placed into said cavity prior to casting the tubular handle portion on the dental instrument.

## Patentansprüche

1. Zahnärztliches Instrument umfassend:
- einen länglichen Handgriffteil (104, 204) und operative Teile (101a, 101b) an den Enden des Handgriffteils (104, 204) für Arbeitsgänge nach einem oder mehreren Anwendungszwecken des zahnärztlichen Instruments und
- einen Körperteil (103, 203) zwischen den operativen Teilen (101a, 101b), der sich mechanisch an die operativen Teile (101a, 101b) anschließt und mindestens teilweise von dem Handgriffteil (104, 204) bedeckt ist,
wobei das zahnärztliche Instrument ferner einen Radiofrequenzidentifikator (106, 206), der aus einer Entfernung des zahnärztlichen Instruments lesbar ist, so umfasst, dass der Radiofrequenzidentifikator (106, 206) von dem Handgriffteil (104, 204) bedeckt ist, wobei der Handgriffteil (104, 204) am Radiofrequenzidentifikator (106, 206) und dem Körperteil (103, 203) angegossen ist, sodass Material des rohrförmigen Handgriffteils (104, 204) am Material des Körperteils (103, 203) haftet, wodurch der Radiofrequenzidentifikator auf dem Körperteil (103, 203) und zwischen dem rohrförmigen Handgriffteil (104, 204) und dem Körperteil (103, 203) positioniert ist, **dadurch gekennzeichnet, dass** der Körperteil aus Metall besteht und eine Länge des zahnärztlichen Instruments (L1) ungefähr 146 - 210 mm beträgt, und der Radiofrequenzidentifikator (106, 206) eingerichtet ist, um eine Wellenlänge von 300 mm zu verwenden.

2. Zahnärztliches Instrument nach Anspruch 1, wobei der spezifische elektrische Widerstand des Materials des rohrförmigen Handgriffteils mindestens 10⁻³ Ωm bei 20 °C beträgt.

3. Zahnärztliches Instrument nach Anspruch 1, wobei das Material des rohrförmigen Handgriffteils elektrisch nichtleitend ist.

4. Zahnärztliches Instrument nach einem der Ansprüche 1 - 3, wobei das Material des rohrförmigen Handgriffteils mechanisch weicher ist als das Material des Körperteils und der Körperteil einen mechanischen Stützkern des zahnärztlichen Instruments bildet.

5. Zahnärztliches Instrument nach einem der Ansprüche 1 - 4, wobei der Elastizitätsmodul des Materials des Körperteils mindestens 1000 MPa beträgt.

6. Zahnärztliches Instrument nach einem der Ansprüche 1 - 5, wobei das Material des rohrförmigen Handgriffteils Silicon oder ein anderes Elastomer ist.

7. Zahnärztliches Instrument nach einem der Ansprüche 1 - 6, wobei der Körperteil (203) einen Hohlraum für den Radiofrequenzidentifikator umfasst.

8. Zahnärztliches Instrument nach Anspruch 6, wobei das Material des Körperteils eins der folgenden ist: Aluminium, Stahl, Polyetheretherketon "PEEK", Polyphenylensulfid "PPS", Polysulfon "PSU", Polyamid "PPSU", Polyetherimid "PEI", Polyamid "PA", Polypropen "PP", Polycarbonat "PC".

9. Verfahren zur Herstellung eines zahnärztlichen Instruments, das einen länglichen Handgriffteil (104, 204) und operative Teile (101a, 101b) an den Enden des Handgriffteils (104, 204) für Arbeitsgänge nach einem oder mehreren Anwendungszwecken des zahnärztlichen Instruments und einen Körperteil (103, 203) zwischen den operativen Teilen (101a, 101b) umfasst, der sich mechanisch an die operativen Teile (101a, 101b) anschließt und mindestens teilweise von dem Handgriffteil (104, 204) bedeckt ist, wobei das Verfahren Folgendes umfasst:
- Platzieren (301) eines Radiofrequenzidentifikators (106, 206) auf den Körperteil des zahnärztlichen Instruments durch Bereitstellen (302) eines rohrförmigen Handgriffteils, um den Radiofrequenzidentifikator und mindestens einen Teil des Körperteils durch Angießen des rohrförmigen Handgriffteils an den Radiofrequenzidentifikator und den Körperteil zu umgeben, sodass Material des rohrförmigen Handgriffteils am Material des Körperteils haftet, und sodass der Radiofrequenzidentifikator zwischen dem rohrförmigen Handgriffteil und dem Körperteil nach Gießen positioniert ist,
**dadurch gekennzeichnet, dass** der Körperteil aus Metall besteht und eine Länge des zahnärztlichen Instruments (L1) ungefähr 146 mm - 210 mm beträgt und der Radiofrequenzidentifikator (106, 206) eingerichtet ist, eine Wellenlänge von 300 mm zu verwenden.

10. Verfahren nach Anspruch 9, wobei Platzieren des Radiofrequenzidentifikators auf den Körperteil des zahnärztlichen Instruments Befestigen des Radiofrequenzidentifikators an einer Fläche des Körperteils mit Klebematerial umfasst.

11. Verfahren nach Anspruch 10, wobei der Körperteil des zahnärztlichen Instruments eingerichtet ist, einen Hohlraum mit Abmessungen zu umfassen, die denjenigen des Radiofrequenzidentifikators entsprechen, und der Radiofrequenzidentifikator in den Hohlraum gesetzt wird, ehe der rohrförmige Handgriffteil an das zahnärztliche Instrument angegossen wird.

## Revendications

1. Instrument dentaire comprenant :
- une partie poignée allongée (104, 204) et des parties opératoires (101a, 101b) au niveau des extrémités de la partie poignée (104, 204) destinées à des opérations conformes à un but d'utilisation de l'instrument dentaire ou plus, et
- une partie corps (103, 203) entre les parties opératoires (101a, 101b) reliant mécaniquement les parties opératoires (101a, 101b) et au moins partiellement couverte par la partie poignée (104, 204),
dans lequel l'instrument dentaire comprend en outre un identificateur radiofréquence (106, 206) qui peut être lu à distance de l'instrument dentaire de telle sorte que l'identificateur radiofréquence (106, 206) est couvert par ladite partie poignée (104, 204), la partie poignée (104, 204) étant coulée sur l'identificateur radiofréquence (106, 206) et la partie corps (103, 203) de sorte que le matériau de la partie poignée tubulaire (104, 204) adhère au matériau de la partie corps (103, 203), l'identificateur radiofréquence étant ainsi positionné sur la partie corps (103, 203) et entre la partie poignée tubulaire (104, 204) et la partie corps (103, 203), **caractérisé en ce que** la partie corps est en métal et une longueur de l'instrument dentaire (L1) est d'environ 146 mm à 210 mm, et l'identificateur radiofréquence (106, 206) est agencé pour utiliser une longueur d'onde de 300 mm.

2. Instrument dentaire selon la revendication 1, dans lequel la résistivité électrique du matériau de la partie poignée tubulaire est d'au moins 10⁻³ Ωm à 20 °C.

3. Instrument dentaire selon la revendication 1, dans lequel le matériau de la partie poignée tubulaire n'est pas électroconducteur.

4. Instrument dentaire selon l'une quelconque des revendications 1 à 3, dans lequel le matériau de la partie poignée tubulaire est mécaniquement plus souple que le matériau de la partie corps et la partie corps constitue un noyau de support mécanique de l'instrument dentaire.

5. Instrument dentaire selon l'une quelconque des revendications 1 à 4, dans lequel le module d'élasticité du matériau de la partie corps est d'au moins 1 000 MPa.

6. Instrument dentaire selon l'une quelconque des revendications 1 à 5, dans lequel le matériau de la partie poignée tubulaire est la silicone ou un autre élastomère.

7. Instrument dentaire selon l'une quelconque des revendications 1 à 6, dans lequel la partie corps (203) comprend une cavité pour l'identificateur radiofréquence.

8. Instrument dentaire selon la revendication 6, dans lequel le matériau de la partie corps est l'un des suivants : aluminium, acier, polyéther-éther-cétone « PEEK », sulfure de polyphénylène « PPS », polysulfone « PSU », polyamide « PPSU », polyétherimide « PEI », polyamide « PA », polypropène « PP », polycarbonate « PC ».

9. Procédé de fabrication d'un instrument dentaire comprenant une partie poignée allongée (104, 204) et des parties opératoires (101a, 101b) au niveau des extrémités de la partie poignée (104, 204) destinées à des opérations conformes à un but d'utilisation de l'instrument dentaire ou plus, et une partie corps (103, 203) entre les parties opératoires (101a, 101b) reliant mécaniquement les parties opératoires (101a, 101b) et au moins partiellement couverte par la partie poignée (104, 204), le procédé comprenant :
- le positionnement (301) d'un identificateur radiofréquence (106, 206) sur la partie corps de l'instrument dentaire en fournissant (302) une partie poignée tubulaire pour entourer l'identificateur radiofréquence et au moins une partie de la partie corps en coulant la partie poignée tubulaire sur l'identificateur radiofréquence et la partie corps de telle sorte que le matériau de la partie poignée tubulaire adhère au matériau de la partie corps et de telle sorte que l'identificateur radiofréquence est positionné entre la partie poignée tubulaire et la partie corps après le coulage,
**caractérisé en ce que** la partie corps est en métal et une longueur de l'instrument dentaire (L1) est d'environ 146 mm à 210 mm, et l'identificateur radiofréquence (106, 206) est agencé pour utiliser une longueur d'onde de 300 mm.

10. Procédé selon la revendication 9, dans lequel le positionnement de l'identificateur radiofréquence sur la partie corps de l'instrument dentaire comprend la fixation de l'identificateur radiofréquence à une surface sur la partie corps avec un matériau adhésif.

11. Procédé selon la revendication 10, dans lequel la partie corps de l'instrument dentaire est agencée pour comprendre une cavité avec des dimensions correspondant à celles de l'identificateur radiofréquence et l'identificateur radiofréquence est placé dans ladite cavité avant de couler la partie poignée tubulaire sur l'instrument dentaire.
